# FASCICULE DE BREVET EUROPEEN

(11) **EP 1 240 177 B1**
(45) Date de publication et mention de la délivrance du brevet: **02.07.2003**
(21) Numéro de dépôt: 00988885.0
(22) Date de dépôt: 06.12.2000
(51) Int. Cl.: C07H 15/203, A61K 31/70, A61P 9/10

(54) **BENZOPHENONE ALPHA-D-GLYCOPYRANOSIDES, PREPARATION ET UTILISATION EN THERAPEUTIQUE**
BENZOPHENON ALPHA-GLYCOPYRANOSIDE, HERSTELLUNG UND THERAPEUTISCHE VERWENDUNG
BENZOPHENONE ALPHA-D-GLYCOPYRANOSIDES, PREPARATION AND THERAPEUTIC USE

(30) Priorité: 23.12.1999 FR 9916387
(43) Date de publication de la demande: 18.09.2002
(73) Titulaire: LABORATOIRES FOURNIER S.A., 21100 Dijon (FR)
(72) Inventeur: LEBRETON, Luc, F-21000 Dijon (FR); LEGENDRE, Christiane, F-21370 Velars-sur-Ouche (FR); SAMRETH, Soth, F-21121 Daix (FR)
(74) Mandataire: Gillard, Marie-Louise
(86) Numéro de dépôt international: FR0003420
(87) Numéro de publication internationale: WO01047941

(56) Documents cités:
- EP-A- 0 346 943
- WO-A-99/67261
- US-A- 4 432 973
- F BELLAMY ET AL: "Glycosylated Derivatives of Benzophenone, Benzhydrol, and Benzhydril as Potential Venous Antithrombotic Agents" JOURNAL OF MEDICINAL CHEMISTRY,US,AMERICAN CHEMICAL SOCIETY. WASHINGTON, vol. 36, no. 7, 2 avril 1993 (1993-04-02), pages 898-903, XP002111914 ISSN: 0022-2623 cité dans la demande

## Description

### Domaine de l'invention

La présente invention concerne, en tant que produits industriels nouveaux, des dérivés de la 4-cyano-4'-hydroxybenzophénone de formule I ci-après qui sont des benzophénone α-D-glycopyranosides. Elle concerne également leur procédé de préparation ainsi que leur utilisation en thérapeutique, notamment sous forme de compositions les contenant en tant que principes actifs.

### Art antérieur

On connaît de EP-A-0051023 des composés présentant un reste hydroxybenzophénone substitué par un groupe β-D-xylosyle, et ayant une activité pharmacologique intéressante pour traiter ou prévenir les thromboses veineuses.

On connaît également de EP-A-0133103, des dérivés du type benzylphényl β-D-xyloside doués de propriétés hypocholestérolémiantes et hypolipidémiantes. On sait aussi que dans EP-A-0365397, EP-A-0290321 ont été décrits des dérivés dans lesquels le radical β-D-xylosyle a été remplacé par un radical β-D-thioxylosyle, lesdits composés étant utiles en raison de leur activité antithrombotique.

On connaît enfin de l'article de F. BELLAMY *et al*., J. Med. Chem., 1993, 36 (No 7) pages 898-903 des composés dérivés de benzophénone substitués par des groupements glycosyle, parmi lesquels seuls les dérivés de configuration β présentent une activité antithrombotique. L'étude de ces produits a démontré que ces composés, et particulièrement ceux comportant un groupement β-D-xylosyle, étaient de bons substrats de la galactosyltransférase I et, en conséquence, étaient capables d'initier la synthèse des glycosaminoglycanes (GAGs). Ce mode d'action, obtenu après administration du produit par voie orale, est très probablement responsable de l'activité antithrombotique et seuls les dérivés de configuration β du D-xylose présentent une activité dans ce domaine thérapeutique. Il existe donc une corrélation entre l'action sur la synthèse des GAGs et l'activité antithrombotique qui rendait les composés autres que ceux dérivés du β-D-xylose sans intérêt dans ce domaine thérapeutique.

### But de l'invention

Selon l'invention, on se propose de fournir une nouvelle solution technique permettant d'aboutir à des produits nouveaux thérapeutiquement intéressants vis-à-vis des plaques athéromateuses artérielles, soit pour traiter lesdites plaques, soit pour prévenir leur apparition.

### Objet de l'invention

Selon la nouvelle solution technique de l'invention, on fait appel à des composés [4-(4-cyanobenzoyl)phényl] α-D-glycopyranosides qui, de façon surprenante eu égard aux publications précédemment citées, présentent une activité dans la prévention ou la régression des plaques athéromateuses artérielles.

Les nouveaux produits selon l'invention sont caractérisés en ce qu'ils sont choisis parmi l'ensemble constitué par :
(i) les [4-(4-cyanobenzoyl)phényl] α-D-glycopyranosides de formule I : 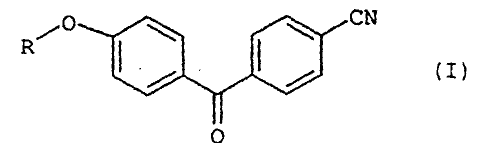 dans laquelle le groupe α-D-glycopyranosyle R représente un groupe α-D-glucopyranosyle, α-D-galactopyranosyle, α-D-mannopyranosyle, α-D-arabinopyranosyle, α-D-lyxopyranosyle, ou α-D-ribopyranosyle ; et,
(ii) leurs esters résultant de l'estérification d'au moins d'une fonction OH de chaque groupe glycopyranosyle par un acide alcanoïque ou cycloalcanoïque en C₂-C₄.

Selon un second aspect de l'invention, on propose un procédé de préparation des composés de formule I ci-dessus et de leurs esters.

Selon encore un troisième aspect de l'invention, on fournit une composition thérapeutique caractérisée en ce qu'elle renferme, en association avec un excipient physiologiquement acceptable, une quantité thérapeutiquement efficace d'au moins un composé de formule I ou de l'un de ses esters.

Selon un autre aspect de l'invention, on préconise également l'utilisation d'un composé de formule I ou de l'un de ses esters en tant que principe actif pour l'obtention d'un médicament destiné à un usage en thérapeutique vis-à-vis de la plaque d'athérome, en particulier pour la prévention ou le traitement de celle-ci.

### Description détaillée

Les nouveaux composés selon l'invention comprennent les produits de la formule I et leurs esters ; ils sont des dérivés pyranosides de 4-cyano-4'-hydroxybenzophénone [ou 4-(4-hydroxybenzoyl)benzonitrile]. Les produits préférés, dans lesquels le radical glycoside est sous forme pyranose, répondent aux formules suivantes données en fonction de la structure du groupe glycopyranosyle R de configuration α-D :

### (a) structure α-D-glucose : (α-D-Glc) :

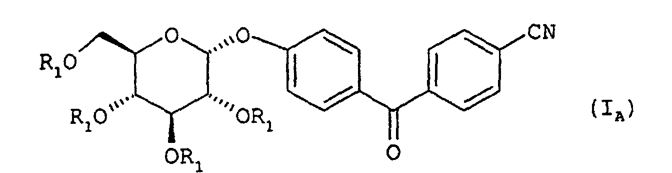

### (b) structure α-D-galactose : (α-D-Gal) :

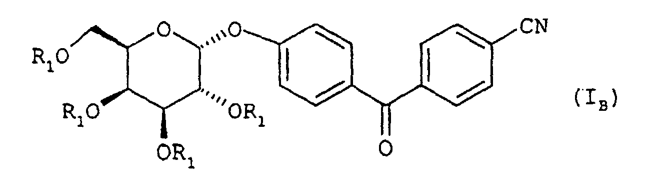

### (c) structure α-D-mannose : (α-D-Man) :

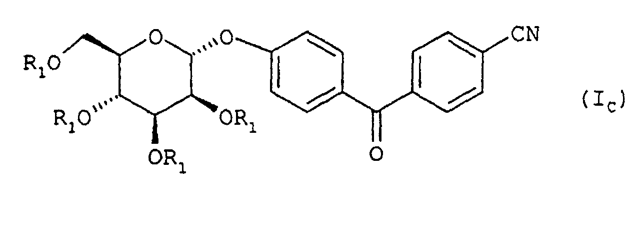

### (d) structure α-D-arabinose : (α-D-Ara) :

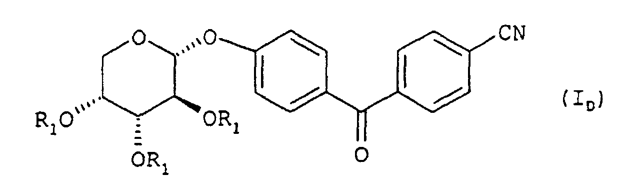

### (e) structure α-D-lyxose : (α-D-Lyx) :

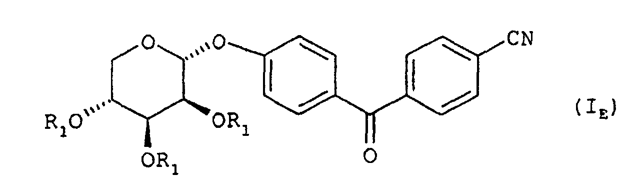

### (f) structure α-D-ribose : (α-D-Rib) :

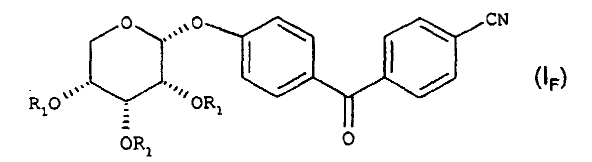

Dans ces formules, R₁ représente un atome d'hydrogène ou un groupe COR₂, R₂ étant un groupe alkyle en C₁-C₃ choisi parmi les groupes méthyle, éthyle, propyle, isopropyle et cyclopropyle.

Le procédé de préparation d'un composé de formule I ou de l'un de ses esters selon l'invention est caractérisé en ce qu'il comprend :
(1°) la réaction d'un pentose ou hexose peracétylé de structure pyranosyle, répondant à la formule II : 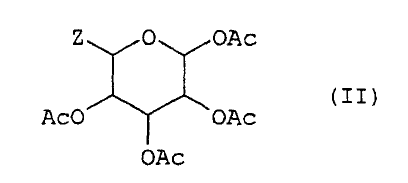 où Z est H ou CH₂OAc,
   et choisi parmi l'ensemble constitué par les 1,2,3,4,6-pentaacétyl-D-glucose, 1,2,3,4,6-pentaacétyl-D-galactose, 1,2,3,4,6-pentaacétyl-D-mannose, 1,2,3,4-tétraacétyl-D-arabinose, 1,2,3,4-tétraacétyl-D-lyxose et 1,2,3,4-tétraacétyl-D-ribose,
   avec le 4-(4-hydroxybenzoyl)benzonitrile de formule III : 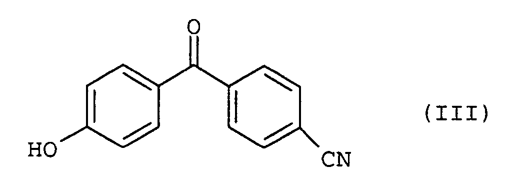 pour obtenir après purification le composé oside correspondant de formule IV 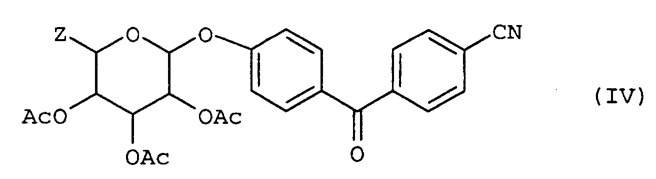 où Z est défini comme indiqué ci-dessus ; et,
(2°) si nécessaire, la réaction de déplacement des groupes acétyle du composé oside de formule IV, ainsi obtenu, pour les remplacer par des atomes d'hydrogène et obtenir le composé de formule I correspondant, les autres esters pouvant être obtenus par estérification dudit composé de formule I avec un acide en C₃-C₄.

De façon avantageuse, la réaction II + III de l'étape (1°) est effectuée dans un solvant organique, (notamment le dichlorométhane), en présence d'un acide de Lewis (tel que par exemple le tétrachlorure d'étain), à une température comprise entre 25°C et la température d'ébullition du solvant, pendant 10 à 30 heures.

A l'étape (2°) le remplacement des groupements Ac par des atomes d'hydrogène est avantageusement mené comme suite. On fait réagir le composé de formule IV avec NH₃ en solution dans un alcool anhydre (notamment le méthanol) pour déplacer les groupements Ac et les remplacer par H.

En variante, la réaction II + III → IV de l'étape (1°) peut être remplacée par la réaction V + III → IV, où V est un halogénopentose ou un halogénohexose peracétylé correspondant. Dans cette circonstance l'étape (1°) devient l'étape (1') qui suit, à savoir :
(1') la réaction d'un halogénopentose ou halogénohexose peracétylé de structure pyranosyle, répondant à la formule V : 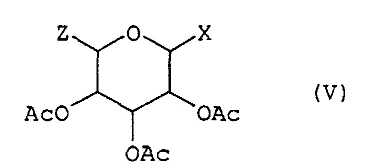 où X est un atome d'halogène (i.e. F, Cl, Br ou I, l'atome d'halogène préféré étant Br), et Z est H ou CH₂OAc,
   et choisi parmi l'ensemble constitué par les 1-bromo-2,3,4,6-tétraacétyl-D-glucose, 1-bromo-2,3,4,6-tétraacétyl-D-galactose, 1-bromo-2,3,4,6-tetraacétyl-D-mannose, 1-bromo-2,3,4-triacétyl-D-arabinose, 1-bromo-2,3,4-triacétyl-D-lyxose, 1-bromo-2,3,4-triacétyl-D-ribose,
   avec le 4-(4-hydroxybenzoyl)benzonitrile de formule III : 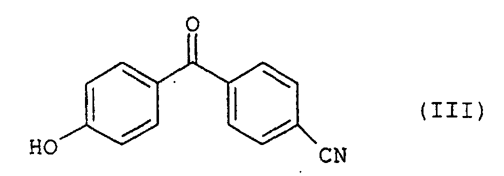 pour obtenir après purification le composé oside correspondant de formule IV. 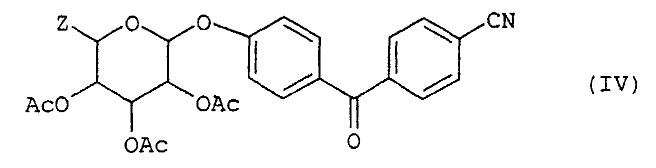 où Z est défini comme indiqué ci-dessus.

De façon avantageuse, la réaction V + III → IV est réalisée dans un solvant anhydre tel que le dichlorométhane, 1-2-dichloroéthane ou l'acétonitrile, en présence d'un agent de couplage tel que le trifluorométhanesulfonate d'argent ou l'oxyde d'argent, à une température de l'ordre de -10 à + 10°C, pendant 5 à 40 heures.

Les réactions II + III → IV et V + III → IV s'appliquent à la préparation de l'ensemble des composés de formule IV, selon l'invention.

D'autres avantages et caractéristiques de l'invention seront mieux compris à la lecture qui va suivre d'exemples de préparation et d'essais pharmacologiques. Bien entendu, l'ensemble de ces éléments n'est pas limitatif mais est fourni à titre d'illustration.

### Exemple 1 (Formule I_{A}, Rₗ=COCH₃) [4-[4-cyanobenzoyl)phényl] 2,3,4,6-tétra-O-acétyl-α-D-glucopyranoside

On prépare une suspension de 12,17 g (31.10⁻³ mole) de 1,2,3,4,6-penta-O-acétyl-β-D-glucose et 10,36 g (46.10⁻³ mole) de 4-(4-hydroxybenzoyl)benzonitrile dans 500 ml de dichlorométhane et on ajoute progressivement, sous agitation et à 0°C, 8,5 ml (72,6.10⁻³ mole) de tétrachlorure d'étain anhydre. Après 20 heures sous agitation à température ambiante, on ajoute à nouveau 4,25 ml (36,3.10⁻³ mole) de tétrachlorure d'étain anhydre et on porte le mélange réactionnel à doux reflux pendant 24 heures. Après refroidissement, le milieu réactionnel est versé sur de la glace. La phase organique séparée est ensuite lavée à l'eau, extraite par une solution de soude 1N, puis lavée à l'eau jusqu'à neutralité et séchée sur sulfate de magnésium. Après concentration sous pression réduite, le résidu est purifié par chromatographie sur gel de silice en éluant à l'aide d'un mélange toluène/acétate d'éthyle (8/2 ; v/v). On obtient ainsi 918 mg du produit attendu sous forme d'un solide blanc amorphe (rendement = 5,3 %).
F = 63°C
[α]_{D}²⁹ = + 83,2° (c = 0,25 ; DMSO)

### Exemple 2 (Formule I_{A}, Rₗ=H) [4-(4-cyanobenzoyl)phényl] α-D-glucopyranoside

On prépare une solution de 575 mg (1,06.10⁻³ mole) du composé obtenu selon l'exemple 1 dans 50 ml de méthanol et on ajoute, à 0°C et sous agitation, 5,6 ml d'une solution saturée d'ammoniac dans le méthanol. On maintient ensuite le milieu réactionnel sous agitation pendant 6 heures à température ambiante puis on chasse le solvant sous pression réduite. Le produit brut obtenu est purifié par chromatographie sur gel de silice en éluant à l'aide d'un mélange dichlorométhane/méthanol (9/1 ; v/v). On obtient ainsi 137 mg du produit attendu sous forme d'un solide fin blanc (rendement = 34 %).
F = 130°C
[α]_{D}²³ = + 145° (c = 0,33 ; DMSO)

### Exemple 3 (Formule I_{C}, Rₗ=COCH₃) [4-(4-cyanobenzoyl)phényl] 2,3,4,6-tétra-O-acétyl-α-D-mannopyranoside

On prépare une solution de 9,9 g (144.10⁻³ mole) de [4-(4-hydroxybenzoyl)benzonitrile et 26 g (63.10⁻³ mole) de bromure de 2,3,4,6-tétra-O-acétyl-α-D-mannopyranosyle dans 300 ml de 1-2 dichloréthane, en présence d'environ 4 g de tamis moléculaire. Le mélange est refroidi à -20°C et on ajoute, à cette température, 34 g (132.10⁻³ mole) de trifluorométhanesulfonate d'argent. Le mélange réactionnel est maintenu sous agitation pendant 24 heures à 0°C, puis est filtré pour éliminer les particules solides. La phase organique est lavée à l'aide d'une solution d'acide chlorhydrique diluée, puis à l'eau, puis avec une solution de soude diluée et enfin à l'eau. Après séchage sur sulfate de magnésium, la solution est concentrée sous pression réduite et le produit brut obtenu est purifié par chromatographie sur gel de silice en éluant à l'aide d'un mélange toluène/acétate d'éthyle. On obtient ainsi 19 g du produit attendu sous forme d'un solide jaune pâle (rendement = 77 %).
F = 60°C
[α]_{D}²⁷ = + 64° (c = 0,62 ; DMSO)

### Exemple 4 (Formule I_{C}, Rₗ=H) [4-(4-cyanobenzoyl)phényl] α-D-mannopyranoside

On dissout 16,7 g (30.10⁻³ mole) du produit obtenu selon l'exemple 3 dans 50 ml de méthanol et on ajoute, à 0°C, 100 ml d'une solution saturée d'ammoniac dans le méthanol. Le mélange réactionnel est agité pendant 6 heures à 0-10°C, puis concentré sous pression réduite. Le produit brut est purifié par chromatographie sur gel de silice en éluant à l'aide d'un mélange dichlorométhane/méthanol (15/1 ; v/v). La fraction de produit pur est cristallisée dans l'acétone et on obtient 7,9 g du produit attendu sous forme de fins cristaux beige clair (rendement = 68 %).
F = 145°C
[α]_{D}²⁷ = + 102° (c = 0,17 ; DMSO)

### Exemple 5 (Formule I_{B}, Rₗ=COCH₃) [4-(4-cyanobenzoyl)phényl] 2,3,4,6-tétra-O-acétyl-α-D-galactopyranoside

En opérant de façon analogue à l'exemple 1, au départ du 1,2,3,4,6-penta-O-acétyl-D-galactose, on obtient le produit attendu sous forme d'un solide amorphe avec un rendement de 4 %.
F = 64-66°C
[α]_{D}²⁹ = + 156° (c = 0,26 ; DMSO)

### Exemple 6 (Formule I_{B}, Rₗ=H) [4-(4-cyanobenzoyl)phényl] α-D-galactopyranoside

En opérant de façon analogue à l'exemple 2, au départ du composé obtenu selon l'exemple 5, on obtient le produit attendu sous forme d'un solide blanc avec un rendement de 90 %.
F = 240°C
[α]_{D}²⁹ = + 173° (c = 0,25 ; DMSO)

### Exemple 7 (Formule I_{D}, Rₗ=COCH₃) [4-(4-cyanobenzoyl)phényl] 2,3,4-tri-O-acétyl-α-D-arabinopyranoside

En opérant de façon analogue à l'exemple 3, au départ du bromure de 2,3,4-tri-O-acétyl-D-arabinopyranosyle, on obtient le produit attendu sous forme d'une huile jaune avec un rendement de 15 %.
[α]_{D}²⁴ = - 10,7° (c = 0,38 ; CH₂Cl₂)

### Exemple 8 (Formule I_{D}, Rₗ=H) [4-(4-cyanobenzoyl)phényl] α-D-arabinopyranoside

En opérant de façon analogue à l'exemple 4, au départ du composé obtenu selon l'exemple 7, on obtient le produit attendu sous forme d'un solide fin beige avec un rendement de 75 %.
F = 160°C
[α]_{D}²⁶ = - 66° (c = 0,32 ; DMSO)

### Exemple 9 (Formule I_{E}, Rₗ=COCH₃) f4-(4-cyanobenzoyl)phényl] 2,3,4-tri-O-acétyl-α-D-lyxopyranoside

En opérant de façon analogue à l'exemple 1, au départ du 1,2,3,4-tétra-O-acétyl-D-lyxopyranose, on obtient le produit attendu sous forme d'une huile avec un rendement de 60 %.
[α]_{D}²⁴ = + 40,3° (c = 0,67 ; CH₂Cl₂)

### Exemple 10 (Formule I_{E}, Rₗ=H) [4-(4-cyanobenzoyl)phényl] α-D-lyxopyranoside

En opérant de façon analogue à l'exemple 2, au départ du composé obtenu selon l'exemple 9, on obtient le produit attendu sous forme d'un solide blanc avec un rendement de 90 %.
F = 173°C
[α]_{D}²⁸ = + 125° (c = 0,175 ; DMSO)

### Exemple 11 (Formule I_{F}, Rₗ=H) [4-(4-cyanobenzoyl)phényl] α-D-ribopyranoside

En opérant de façon analogue à l'exemple 1, au départ du 1,2,3,4-tetra-O-acétylribopyranose, on obtient le [4-(4-cyanobenzoyl)-phényl] 2,3,4-tri-O-acétyl-α-D-ribopyranoside sous forme d'un solide jaune que l'on traite à l'aide d'une solution d'ammoniac dans le méthanol, selon le protocole décrit à l'exemple 2, pour obtenir le produit attendu sous forme d'une poudre blanche avec un rendement global de 6%.
F = 164°C
[α]_{D}²⁶ = + 77,7° (c = 0,21 ; DMSO)

L'activité antiathéromateuse des composés selon l'invention a été évaluée en fonction de leur aptitude à abaisser le taux de cholestérol sérique chez des souris soumises à un régime gras. Il a en effet été démontré dans plusieurs publications une corrélation étroite entre une surcharge lipidique et une augmentation marquée du risque athéromateux (cf. Lancet 1996, 348 pages 1339-1342 ; Lancet 1990 ; 335 pages 1233-1235). Cette corrélation permet de mettre en oeuvre un test plus rapide que les essais directs sur la plaque d'athérome, lesdits essais nécessitant un traitement long des animaux et une étude histologique lourde des parois de la crosse aortique.

Le test mis en oeuvre consiste à administrer une dose unique du composé à des souris femelles de souche C57BL/6J. Le protocole est le suivant : le premier jour (J0), les souris sont mises à jeun de 9 à 17 heures, un prélèvement sanguin étant effectué à 14 heures. A 17 heures, une quantité déterminée de nourriture (régime gras comprenant 1,25 % de cholestérol et 0,5 % d'acide cholique) est distribuée. Le second jour (J1), à 9 heures, les restes de nourriture sont pesés et les souris mises à jeun de 9 à 14 heures. A 14 heures, un prélèvement sanguin est effectué. Pour les groupes de souris traitées, le composé est administré par tubage, en suspension dans une solution d'eau gommeuse, à 3 %, le second jour (J1) à 9 heures. Les groupes témoins reçoivent seulement de l'eau gommeuse.

Les composés ont été testés à la dose de 100 mg/kg. Le cholestérol total sérique est dosé et les résultats sont exprimés en pourcentage d'inhibition de l'augmentation de la cholestérolémie par rapport au groupe témoin. Les résultats obtenus sont reportés dans la colonne "Activité" du tableau I. Par ailleurs, on peut noter que l'analyse du contenu en cholestérol des différentes classes de lipoprotéines sériques montre un effet favorable du produit sur le rapport HDL-cholestérol/cholestérol total.

Par ailleurs, on a démontré que les composés de formule I selon l'invention n'induisent pas la synthèse des GAGs.

Les produits de formule I et leurs esters selon l'invention peuvent être administrés, de préférence par voie orale, sous forme de comprimés ou de gélules, renfermant chacun 20 à 500 mg d'un composé de formule I ou l'un de ses esters en tant que principe actif, en association avec des excipients. La posologie sera d'environ 1 à 4 prises par jour. Les produits selon l'invention sont prescrits avantageusement vis-à-vis de la plaque d'athérome, et en particulier pour la prévention ou le traitement du risque athéromateux.

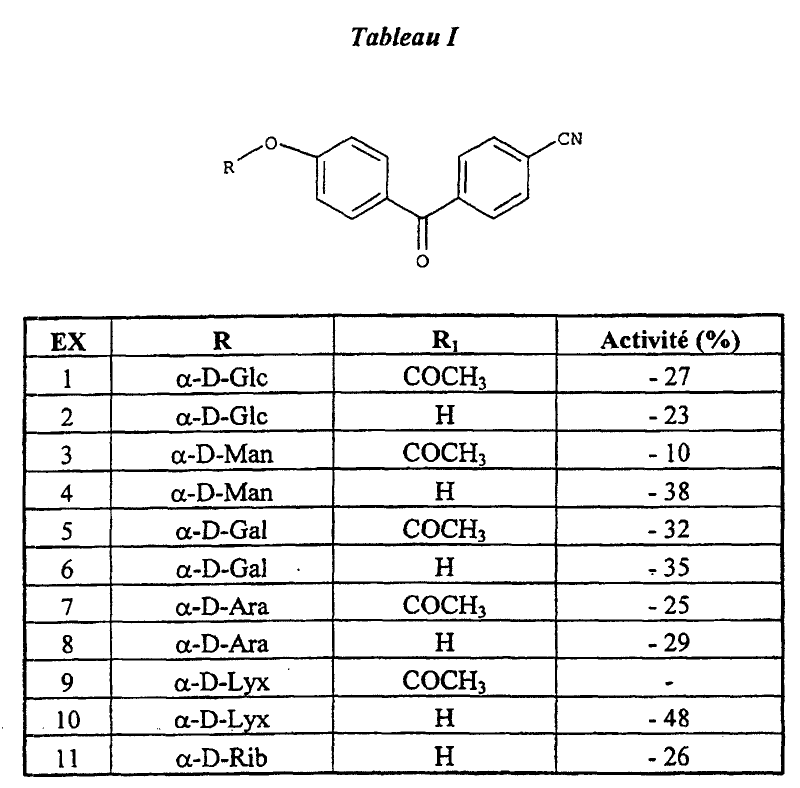

## Revendications

1. Composé α-D-glycopyranoside, **caractérisé en ce qu'**il est choisi parmi l'ensemble constitué par
(i) les [4-(4-cyanobenzoyl)phényl] α-D-glycopyranosides de formule I : 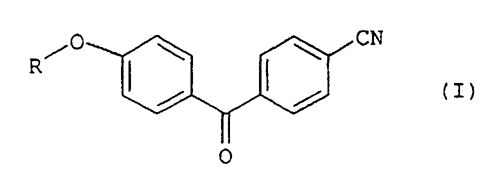 dans laquelle le groupe α-D-glycopyranosyle R représente un groupe α-D-glucopyranosyle, α-D-galactopyranosyle, α-D-mannopyranosyle, α-D-arabinopyranosyle, α-D-lyxopyranosyle, ou α-D-ribopyranosyle ; et,
(ii) leurs esters résultant de l'estérification d'au moins d'une fonction OH de chaque groupe glycopyranosyle par un acide alcanoïque ou cycloalcanoïque en C₂-C₄.

2. [4-(4-cyanobenzoyl)phényl] α-D-glucopyranoside et son dérivé peracétylé.

3. [4-(4-cyanobenzoyl)phényl] α-D-galactopyranoside et son dérivé peracétylé.

4. [4-(4-cyanobenzoyl)phényl] α-D-mannopyranoside et son dérivé peracétylé.

5. [4-(4-cyanobenzoyl)phényl] α-D-arabinopyranoside et son dérivé peracétylé.

6. [4-(4-cyanobenzoyl)phényl] α-D-lyxopyranoside et son dérivé peracétylé.

7. [4-(4-cyanobenzoyl)phényl] α-D-ribopyranoside et son dérivé peracétylé.

8. Composition pharmaceutique **caractérisée en ce qu'**elle renferme, en association avec un excipient physiologiquement acceptable, une quantité thérapeutiquement efficace d'au moins un composé de formule I ou l'un de ses esters selon la revendication 1.

9. Utilisation d'un produit choisi parmi l'ensemble des composés de formule I et leurs esters selon la revendication 1, pour la préparation d'un médicament antiathéromateux destiné à une utilisation en thérapeutique vis-à-vis de la plaque d'athérome.

10. Procédé pour la préparation d'un composé [4-(4-cyanobenzoyl)-phényl] α-D-glycopyranoside de formule I ou de son dérivé peracétylé selon la revendication 1, ledit procédé étant **caractérisé en ce qu'**il comprend :
(1°) la réaction (a) d'un pentose ou hexose peracétylé de structure pyranosyle, répondant à la formule II : 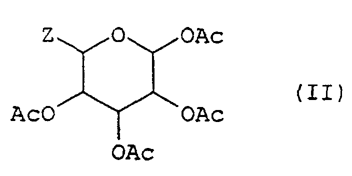 où Z est H ou CH₂OAc,
et choisi parmi l'ensemble constitué par les 1,2,3,4,6-pentaacétyl-D-glucose, 1,2,3,4,6-pentaacétyl-D-galactose, 1,2,3,4,6-pentaacétyl-D-mannose, 1,2,3,4-tétraacétyl-D-arabinose, 1,2,3,4-tétraacétyl-D-lyxose et 1,2,3,4-tétraacétyl-D-ribose,
ou (b) la réaction d'un halogénopentose ou halogénohexose peracétylé de structure pyranosyle, répondant à la formule V : 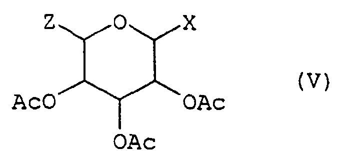 où X est un atome d'halogène (i.e. F, Cl, Br ou I, l'atome d'halogène préféré étant Br), et Z est H ou CH₂OAc,
et choisi parmi l'ensemble constitué par les 1-bromo-2,3,4,6-tétra-acétyl-D-glucose, 1-bromo-2,3,4,6-tétraacétyl-D-galactose, 1-bromo-2,3,4,6-tetraacétyl-D-mannose, 1-bromo-2,3,4-triacétyl-D-arabinose, 1-bromo-2,3,4-triacétyl-D-lyxose, 1-bromo-2,3,4-triacétyl-D-ribose, avec le 4-(4-hydroxybenzoyl)benzonitrile de formule III : 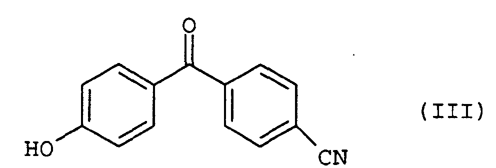 pour obtenir après purification le composé oside correspondant de formule IV 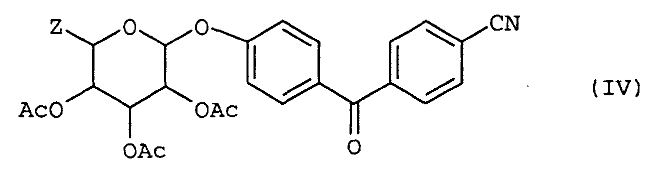 où Z est défini comme indiqué ci-dessus ; et,
(2°) si nécessaire, la réaction de déplacement des groupes acétyle du composé oside de formule IV, ainsi obtenu, pour les remplacer par des atomes d'hydrogène et obtenir le composé de formule I correspondant.

## Patentansprüche

1. α-D-Glycopyranosid-Verbindung, **dadurch gekennzeichnet, dass** sie ausgewählt ist aus
(i) [4-(4-Cyanobenzoyl)phenyl]-α-D-glycopyranosiden der Formel I: 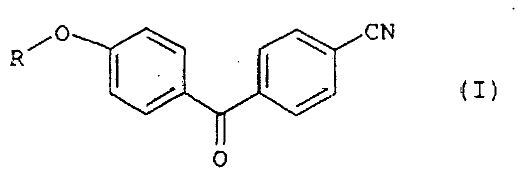 in welcher der α-D-Glycopyranosylrest R einen α-D-Glucopyranosyl-, α-D-Galactopyranosyl-, α-D-Mannopyranosyl-, α-D-Arabinopyranosyl-, α-D-Lyxopyranosyl- oder α-D-Ribopyranosylrest darstellt ; und
(ii) deren Estern, die aus der Veresterung mindestens einer OH-Funktion eines jeden Glycopyranosylrests durch eine C₂-C₄-Alkyl- oder Cycloalkylcarbonsäure resultieren.

2. [4-(4-Cyanobenzoyl)phenyl]-α-D-glucopyranosid und dessen peracetyliertes Derivat.

3. [4-(4-Cyanobenzoyl)phenyl]-α-D-galactopyranosid und dessen peracetyliertes Derivat.

4. [4-(4-Cyanobenzoyl)phenyl]-α-D-mannopyranosid und dessen peracetyliertes Derivat.

5. [4-(4-Cyanobenzoyl)phenyl]-α-D-arabinopyranosid und dessen peracetyliertes Derivat.

6. [4-(4-Cyanobenzoyl)phenyl]-α-D-lyxopyranosid und dessen peracetyliertes Derivat.

7. [4-(4-Cyanobenzoyl)phenyl]-a-D-ribopyranosid und dessen peracetyliertes Derivat.

8. Arzneimittel, **dadurch gekennzeichnet, dass** es eine therapeutisch wirksame Menge mindestens einer Verbindung der Formel I oder eines ihrer Ester gemäß Anspruch 1, in Verbindung mit einem physiologisch verträglichen Excipienten enthält.

9. Verwendung einer Substanz, ausgewählt aus Verbindungen der Formel I und ihren Estern gemäß Anspruch 1, zur Herstellung eines antiatheromatösen Medikaments zur Verwendung in der Therapie von atheromatöser Plaque.

10. Verfahren zur Herstellung einer [4-(4-Cyanobenzoyl)phenyl]-α-D-glycopyranosid-Verbindung der Formel I oder ihres peracetylierten Derivats gemäß Anspruch 1, wobei das Verfahren **dadurch gekennzeichnet ist, dass** es die folgenden Schritte umfasst:
(1) die Reaktion (a) einer peracetylierten Pentose oder Hexose mit Pyranosyl-Struktur der Formel II: 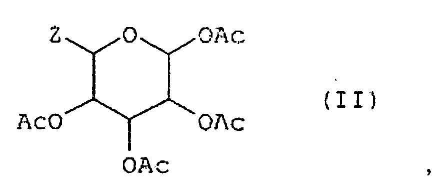 wobei Z ein Wasserstoffatom oder eine Gruppe CH₂OAc ist,
und die peracetylierte Pentose oder Hexose ausgewählt ist aus 1,2,3,4,6-Pentaacetyl-D-glucose, 1,2,3,4,6-Pentaacetyl-D-galactose, 1,2,3,4,6-Pentaacetyl-D-mannose, 1,2,3,4-Tetraacetyl-D-arabinose, 1,2,3,4- Tetraacetyl-D-lyxose und 1,2,3,4- Tetraacetyl-D-ribose,
oder (b) die Reaktion einer peracetylierten Halogenpentose oder Halogenhexose mit Pyranosyl-Struktur der Formel V: 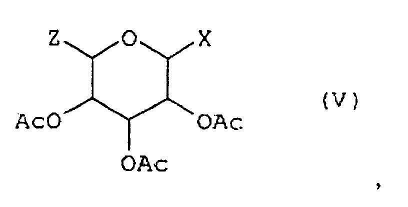 wobei X ein Halogenatom ist (z.B. F, Cl, Br oder I, wobei das Halogenatom bevorzugt Br ist) und Z ein Wasserstoffatom oder eine Gruppe CH₂OAc ist, und die peracetylierte Halogenpentose oder Halogenhexose ausgewählt ist aus 1-Brom-2,3,4,6-tetraacetyl-D-glucose, 1-Brom-2,3,4,6-tetraacetyl-D-galactose, 1-Brom-2,3,4,6-tetraacetyl-D-mannose, 1-Brom-2,3,4-triacetyl-D-arabinose, 1-Brom-2,3,4-triacetyl-D-lyxose, 1-Brom-2,3,4-triacetyl-D-ribose, mit dem 4-(4-Hydroxybenzoyl)benzonitril der Formel III: 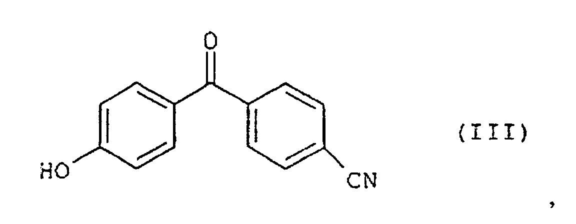 wobei nach Reinigung die entsprechende Osid-Verbindung der Formel IV erhalten wird: 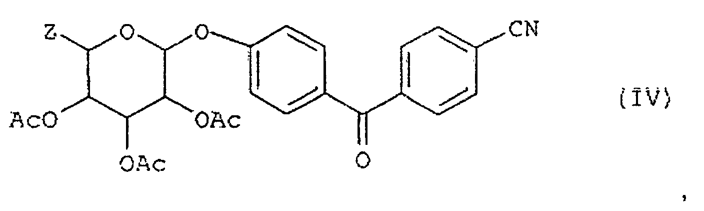 wobei Z die oben angegebene Bedeutung hat; und
(2) gegebenenfalls die Substitutionsreaktion der Acetylgruppen der so erhaltenen Osid-Verbindung der Formel IV um sie durch Wasserstoffatome zu ersetzen und die entsprechende Verbindung der Formel I zu erhalten.

## Claims

1. An α-D-glycopyranoside compound, **characterized in that** it is selected from the group consisting of:
(i) the [4-(4-cyanobenzoyl)phenyl] α-D-glycopyranosides of formula I: 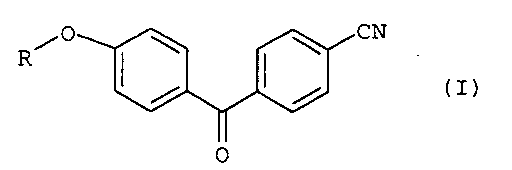 in which the α-D-glycopyranosyl group R is an α-D-glucopyranosyl, α-D-galactopyranosyl, α-D-mannopyranosyl, α-D-arabinopyranosyl, α-D-lyxopyranosyl or α-D-ribopyranosyl group; and
(ii) their esters resulting from the esterification of at least one OH group on each glycopyranosyl group by a C₂-C₄ alkanoic or cycloalkanoic acid.

2. [4-(4-Cyanobenzoyl)phenyl] α-D-glucopyranoside and its peracetylated derivative.

3. [4-(4-Cyanobenzoyl)phenyl] α-D-galactopyranoside and its peracetylated derivative.

4. [4-(4-Cyanobenzoyl)phenyl] α-D-mannopyranoside and its peracetylated derivative.

5. [4-(4-Cyanobenzoyl)phenyl] α-D-arabinopyranoside and its peracetylated derivative.

6. [4-(4-Cyanobenzoyl)phenyl] α-D-lyxopyranoside and its peracetylated derivative.

7. [4-(4-Cyanobenzoyl)phenyl] α-D-ribopyranoside and its peracetylated derivative.

8. A pharmaceutical composition, **characterized in that** it contains, in association with a physiologically acceptable excipient, a therapeutically effective amount of at least one compound of formula I or one of its esters according to claim 1.

9. The use of a product selected from the group of compounds of formula I and their esters according to claim 1 for the preparation of an antiatheromatous drug to be used in therapeutics for combating atheromatous plaque.

10. A process for the preparation of a [4-(4-cyanobenzoyl)phenyl] α-D-glycopyranoside compound of formula I or its peracetylated derivative according to claim 1, said process being **characterized in that** it comprises:
(1°) reacting (a) a peracetylated pentose or hexose of the pyranosyl structure of formula II: 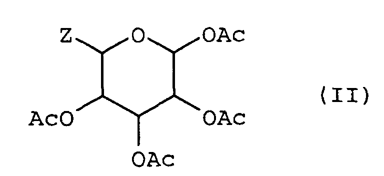 in which Z is H or CH₂OAc,
selected from the group consisting of 1,2,3,4,6-pentaacetyl-D-glucose, 1,2,3,4,6-pentaacetyl-D-galactose, 1,2,3,4,6-pentaacetyl-D-mannose, 1,2,3,4-tetraacetyl-D-arabinose, 1,2,3,4-tetraacetyl-D-lyxose and 1,2,3,4-tetraacetyl-D-ribose,
or (b) a peracetylated halogenopentose or halogenohexose of the pyranosyl structure of formula V: 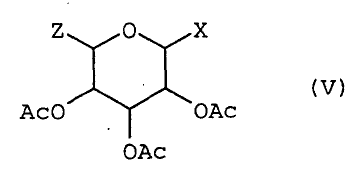 in which X is a halogen atom (i.e. F, Cl, Br or I, the preferred halogen atom being Br) and Z is H or CH₂OAc,
selected from the group consisting of 1-bromo-2,3,4,6-tetraacetyl-D-glucose, 1-bromo-2,3,4,6-tetraacetyl-D-galactose, 1-bromo-2,3,4,6-tetraacetyl-D-mannose, 1-bromo-2,3,4-triacetyl-D-arabinose, 1-bromo-2,3,4-triacetyl-D-lyxose and 1-bromo-2,3,4-triacetyl-D-ribose, with 4-(4-hydroxybenzoyl)benzonitrile of formula III: 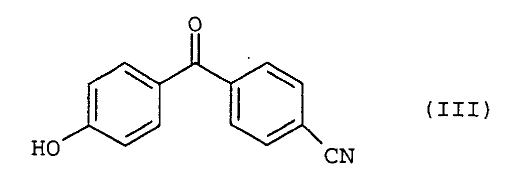 to give, after purification, the corresponding oside compound of formula IV: 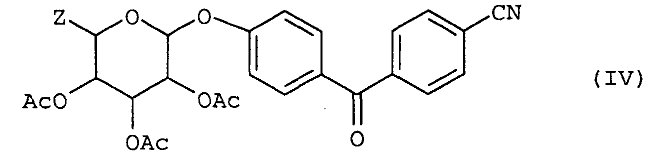 in which Z is defined as indicated above; and
(2°) if necessary, carrying out a displacement reaction on the acetyl groups of the resulting oside compound of formula IV in order to replace them with hydrogen atoms to give the corresponding compound of formula I.
